# EUROPEAN PATENT APPLICATION

(11) **EP 3 842 331 A1**
(43) Date of publication of application: **30.06.2021**
(21) Application number: 19944267.4
(22) Date of filing: 08.11.2019
(51) Int. Cl.: B63C 13/00

(54) **WHEEL STRUCTURE AND CANOE USING THE WHEEL STRUCTURE**

(30) Priority: 02.09.2019 CN 201910821731
(71) Applicant: Ningbo Kayaka Sports Co., Ltd., Zhejiang 315171 (CN)
(72) Inventor: CHEN, Bingrui, Ningbo, Zhejiang 315171 (CN)
(74) Representative: Keil & Schaafhausen Patentanwälte PartGmbB
(86) International application number: PCT/CN2019/116450
(87) International publication number: WO 2021/042506

(57) **Abstract**

The utility model discloses a wheel structure and a kayak using the same, and relates to the field of ships. A main body of the wheel structure is composed of wheel bodies, a shaft and a support rod. A connecting piece is arranged at one end of the support rod relative to the wheel bodies; a bidirectional ratchet wheel mechanism is arranged between the shaft and the support rod; and the bidirectional ratchet wheel mechanism can selectively control the shaft through adjustment, to make the shaft rotate in one direction only, thereby achieving the purpose of controlling the rotation direction of the wheel. The wheel structure is installed on the kayak. When the kayak is loaded, the kayak is easily pushed under the action of a wheel. Meanwhile, the bidirectional ratchet mechanism can limit the rotation direction of the wheel so that the wheel can only roll in the direction of a vehicle. Therefore, a pushing person does not need to powerfully offset the retreating force of the wheel, that is, it is easier and effortless to push and load the kayak.

## Description

### Technical Field

The utility model relates to the field of ships, particularly to a wheel structure and a kayak using the same.

### Background

With the improvement of the living standard, more and more people like the water sports, i.e., kayaking. However, in order to transport a kayak to a designated water area to enter water, the kayak often needs to be loaded onto a vehicle for transport. At present, it is often very difficult to push the kayak onto a vehicle roof manually when loading, and it is very expensive if the kayak is loaded by professional tools, which may increase extra cost.

### Summary

To solve the above technical problem, the purpose of the present invention is to provide a wheel structure capable of achieving loading easily by pushing a kayak manually when loading the kayak.

To achieve the above purpose, the utility model adopts the following technical solution: a wheel structure has a main body composed of wheel bodies, a shaft and a support rod, wherein a bidirectional ratchet wheel mechanism is arranged between the shaft and the support rod.

By adopting the above technical solution, the bidirectional ratchet wheel mechanism can selectively control the shaft through adjustment, to allow the shaft to rotate in one direction, to achieve the purpose of controlling the rotation direction of the wheel. The wheel structure is installed on the kayak. When the kayak is loaded, one end of the kayak is lifted, and the other end is grounded by the wheel; the kayak is pushed in the direction of the vehicle and is easily pushed under the action of the wheel, but it is not enough to rely on the wheel alone because the wheel can roll forwards or roll backwards. In the process of loading the kayak onto the vehicle roof, the weight of the kayak may affect the wheel and make the wheel roll backwards, that is, in the process of loading the kayak onto the vehicle roof, if a pushing person releases hands, the kayak may retreat, so the continuous push of the kayak may actually need to offset the retreating force of the wheel. The bidirectional ratchet mechanism can limit the rotation direction of the wheel so that the wheel can only roll in the direction of the vehicle. Therefore, the pushing person does not need to powerfully offset the retreating force of the wheel, that is, it is easier and effortless to push and load the kayak.

Preferably, the shaft is provided with a retreat preventing rod, and the retreat preventing rod is movably arranged on the shaft.

By adopting the above technical solution, the retreat preventing rod may not swing along with the rotation of the shaft because of being movably arranged on the shaft. When the kayak is pushed and loaded, the kayak is inclined to the ground. At this time, under the action of the ratchet wheel mechanism, the kayak may not slide downwards, and one end of the retreat preventing rod on the shaft comes into contact with the ground and then is abutted against the ground, thereby further limiting the acting force of downward slide of the kayak.

Preferably, the support rod is provided with a camber.

By adopting the above technical solution, if the support rod is a straight rod, when the support rod is turned over to be parallel with a hull of the kayak, the wheel body arranged on the support rod may be fitted with the hull and then may not effectively roll. If the support rod is configured as a bent pipe, when the support rod is turned over to be parallel with the hull, the wheel may not be fitted with the hull and may effectively roll, so that the configured support rod may be applicable to turnover structures and has wider scope of application.

A kayak has a main body provided with any one of the above wheel structures.

Preferably, the kayak is provided with a connecting piece, and the wheel structure is connected with the kayak by the connecting piece.

By adopting the above technical solution, the wheel structure is indirectly disassembled from or installed on the kayak, thereby reducing the possibility of damaging the hull due to frequent disassembly and installation between the wheel structure and the hull.

Preferably, the connecting piece is provided with a turnover structure, and the support rod of the wheel structure is provided with a limiting structure matched with the turnover structure to realize limit.

By adopting the above technical solution, the turnover structure on the connecting piece can make the support rod turn over to realize swing by taking the connecting piece as an end point, and the wheel structure may be perpendicular to the ground or may be parallel to the ground by swinging the support rod. The limiting structure on the support rod is used to match with the turnover structure to realize limit, so that when the support rod is swung to a designated angle through the turnover structure, the operation of matching the limiting structure with the turnover structure can lock the turnover structure, thereby playing a role of fixing the angle of the support rod.

Preferably, the connecting piece comprises a sleeve; the periphery of the sleeve is provided with a plurality of positioning holes; and a spring pin is arranged at one end of the support rod, and the spring pin can be matched with the positioning holes to realize limit.

By adopting the above technical solution, the support rod can rotate at 360° in the sleeve when being inserted into the sleeve. In order to prevent the support rod from rotating during use and keep the support rod structurally stable, after the support rod is inserted into the sleeve, the spring pin is matched with the positioning holes in the sleeve to realize matching limit. Because the sleeve is provided with the plurality of positioning holes, the support rod can be selectively matched with one of the positioning holes to realize limit. Therefore, in the process of using the wheel structure, when a user wants to change the horizontal direction of the wheel, the spring pin is pressed and then retracted; and meanwhile, the support rod is made to rotate so that the support rod rotates in the sleeve. After the wheel rotates to a designated direction, the spring pin is bounced from the positioning hole in the corresponding position to realize matching limit, so that the wheel may be fixed in the direction.

Preferably, the sleeve is detachably provided with a balanced buoy.

By adopting the above technical solution, the support rod of the wheel structure is disassembled from the sleeve and replaced with a balanced buoy, so that the operation of replacement between the wheel structure and the balanced buoy in the process of using the kayak can be realized.

Preferably, the connecting piece is formed by matching a base with the sleeve; the base is arranged on the main body of the kayak; the sleeve is arranged at one end of the support rod; and the sleeve is detachably connected with the base.

By adopting the above technical solution, the sleeve is matched and connected with the base; the sleeve is arranged on the support rod; and the wheel structure is detachably connected with the base on the kayak, so that the operation may be more convenient when the user disassembles or installs the wheel structure.

Preferably, the base and the main body of the kayak are installed by sealing screws.

By adopting the above technical solution, the sealing screws can guarantee the sealing at installing positions to prevent water leakage.

Compared with the prior art, the utility model has the advantages that: (1) the wheel structure is provided with the bidirectional ratchet wheel mechanism which can control the wheel to rotate in one direction only, so that it is easier and effortless when the kayak is loaded; (2) the connecting piece is provided with the turnover structure; when the wheel structure is arranged at the stern of the kayak, the kayak enters water only by turning over the wheel structure without disassembly, so that the operation is more convenient; (3) the connecting piece is provided with the turnover structure; when the wheel structure is arranged at the bottom of the kayak, the wheel structure is made to be perpendicular or parallel to the ground by turning over the wheel structure; the altitudes of the kayak lifted by the wheel structure in two states are different, so that the inclined angle of the kayak can be changed by adjusting the turnover angle of the wheel; (4) by rotating the support rod in the sleeve and selecting matched positioning holes, the rotation adjustment of the wheel structure in the horizontal direction can be realized; and (5) in the process of using the kayak, the replacement of the wheel structure and balanced buoy can be realized.

### Description of Drawings

Fig. 1 is a schematic diagram of a wheel structure of embodiment 1 of the utility model;
Fig. 2 is a schematic diagram of a usage state of embodiment 1 of the utility model;
Fig. 3 is a schematic diagram of a usage state of embodiment 2 of the utility model;
Fig. 4 is a practical schematic diagram of a balanced buoy of embodiment 2 of the utility model;
Fig. 5 is a schematic diagram of a usage state of embodiment 3 of the utility model; and
Fig. 6 is a structural schematic diagram of a connecting piece of embodiment 4 of the utility model.

In the figures: 1. wheel body; 2. shaft; 210. bidirectional ratchet wheel mechanism; 220. retreat preventing rod; 221. U-shaped clip; 222. gravity block; 3. support rod; 310. spring pin; 320. handle; 321. limiting block; 4. connecting piece; 410. sleeve; 411. positioning hole; 420. base; 440. articulated sheet A; 440. articulated sheet B; 450. articulated shaft; 460. limiting groove; 470. sealing screw; 480. chute; 481. sliding block; 482. spring plunger; 5. balanced buoy; 6. kayak.

### Detailed Description

The utility model is further described below in detail in combination with the drawings and embodiments.

### Embodiment 1

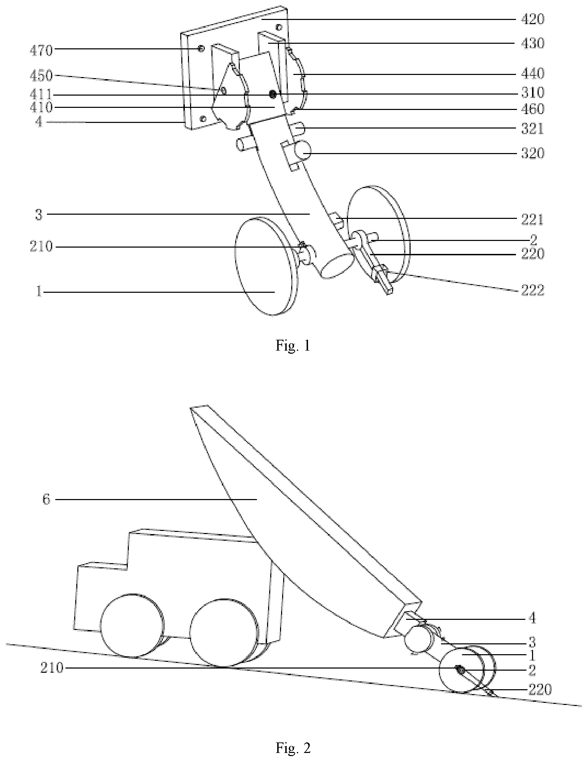

A wheel structure, as shown in Fig. 1, has a main body composed of wheel bodies 1, a shaft 2 and a support rod 3. A bidirectional ratchet wheel mechanism 210 is arranged in the connecting position between the shaft 2 and the support rod 3; a ratchet wheel of the bidirectional ratchet wheel mechanism 210 is fixed to the shaft 2; a ratchet pawl of the ratchet wheel and an adjustment switch for controlling the ratchet pawl are arranged on the support rod 3; under the action of the bidirectional ratchet wheel mechanism 210, the shaft 2 can be adjusted to rotate in one direction only; and then the wheel bodies 1 arranged at both ends of the shaft 2 are adjusted along with the shaft 2 to rotate in one direction only.

In the present embodiment, a retreat preventing rod 220 is arranged on the shaft 2; the retreat preventing rod 220 is movably arranged on the shaft 2, that is, the retreat preventing rod is sleeved on the shaft 2 by a ring at one end; the retreat preventing rod 220 may not swing along with the rotation of the shaft 2; in use, one end of the retreat preventing rod is hung on either side and comes into contact with the ground; in this state, the included angle of the retreat preventing rod 220 close to the side of the wheel bodies 1 is less than 90°, so that the retreat preventing rod 220 is abutted against the ground and the wheel bodies 1 cannot be rotated in the direction of the side where the retreat preventing rod is located, thereby further achieving the effect of making the wheel bodies 1 rotate in one direction only, that is, preventing the wheel bodies 1 from retreating.

In the present embodiment, a gravity block 222 is arranged at the end of the retreat preventing rod 220 coming into contact with the ground; and the gravity block 222 is used to add the weight of the end of the retreat preventing rod 220 coming into contact with the ground, so that the retreat preventing rod 220 becomes steadier when being abutted against the ground, and then is made difficult to bounce when being abutted against the ground.

In the present embodiment, a connecting piece 4 is arranged at the end of the support rod 3 of the wheel structure opposite to the wheel body 1, and the wheel structure is arranged at the stern of the kayak 6 by the connecting piece 4, as shown in Fig. 2. When the kayak 6 is loaded, the user lifts the front end of the kayak 6 to incline the kayak; the kayak is pushed by coming into contact with the ground by the wheel structure located at the stern depending on the rotation of the wheel bodies 1, so that the kayak 6 is pushed more effortlessly; in the process of pushing the kayak 6 onto the vehicle roof, the bidirectional ratchet wheel mechanism 210 achieves the effect of locking the wheel bodies 1 to make the wheel bodies 1 rotate in the forward direction only, thereby preventing the retreat force produced due to downward slide in the process of loading the kayak 6, that is, under the cooperation of the bidirectional ratchet wheel mechanism 210, the kayak 6 is loaded more easily and effortlessly. Meanwhile, when the kayak 6 is pushed and loaded, the retreat preventing rod 220 on the shaft 2 is hung on the side opposite to the vehicle, to make the retreat preventing rod come into contact with the ground to play a role of support; the principle is that: the retreat preventing rod 220 is hung and abutted on the ground on the side opposite to the vehicle, to form an included angle less than 90° with the ground close to the side of the wheel body 1; and if the wheel body 1 rotates backwards, the wheel body 1 may be stopped by the retreat preventing rod 220 and then cannot retreat, thereby further achieving the effect of preventing the kayak 6 from retreating due to downward slide in the process of loading.

In the present embodiment, a U-shaped clip 221 is arranged on one side of the support rod 3, and the U-shaped clip is matched with the retreat preventing rod 220 to realize limit. Thus, the retreat preventing rod 220 may be hung on one side in use, and may be turned up and clamped into a bayonet of the U-shaped clip 221 when not in use, to realize clamping limit, so that the use of the retreat preventing rod 220 is more controllable.

In the present embodiment, the connecting piece 4 is specifically composed of a base 420, a turnover structure arranged on the base 420 and a sleeve 410 connected with the turnover structure. The sleeve 410 is connected with the support rod 3 and can be turned over along with the turnover structure, and the base 420 is connected with the hull of the kayak 6. The sleeve 410 is specifically connected with the support rod 3 in the mode that: one end of the support rod 3 is inserted from one end of the sleeve 410 and connected with the sleeve 410; and because the support rod 3 is a circular pipe and the inner wall of the sleeve 410 is circular, the support rod 3 is rotated in the circumferential direction at 360° in the sleeve 410 after being inserted into and matched with the sleeve 410. The periphery of the sleeve 410 is provided with a plurality of positioning holes 411; and a spring pin 310 is arranged in the position opposite to the support rod 3, so that after the support rod 3 inserted into the sleeve 410, the spring pin 310 is correspondingly bounced from one positioning hole 411 and matched with the positioning hole, thereby playing a role of limiting and fixing the support rod 3 and the sleeve 410. Meanwhile, because the periphery of the sleeve 410 is provided with a plurality of positioning holes 411, the support rod 3 may select any one positioning hole 411 in the horizontal direction to match with the positioning hole. Thus, in use, the user may select a corresponding positioning hole 411 according to an appropriate wheel direction to insert the support rod 3 to match with the positioning hole, or may rotate the support rod 3 in a state of connection with the sleeve 410, that is, the spring pin 310 is pressed and then retracted in the positioning hole 411. Meanwhile, the support rod 3 rotates so as to rotate to the position of a designated positioning hole 411, and then matching is realized by correspondingly bouncing the spring pin 310 from the positioning hole 411. The support rod 3 is rotated in the horizontal direction in the sleeve 410 to select an angle for limiting and fixation, so that the orientation of the wheel structure in the horizontal direction is rotatably adjusted, having higher selectivity.

In the present embodiment, a turnover structure is arranged between the above-mentioned base 420 and sleeve 410; and the sleeve 410 is turned over along with the turnover structure. The specific structure is as follows: two articulated sheets A430 corresponding to each other are arranged on one side of the base 420, the articulated sheets A430 and the base 420 are connected in fixed positions, an articulated shaft 450 is penetrated between the articulated sheets A430, and the articulated shaft 450 can rotate relative to the articulated sheets A430. The sleeve 410 is located between the two articulated sheets A430; the side surfaces thereof are penetrated by the articulated shaft 450 and are fixedly connected with the articulated shaft 450, that is, the sleeve 410 may rotate along with the articulated shaft 450 in the same direction; and in the case where the support rod 3 is inserted into one end of the sleeve 410 and connected with the sleeve 410, through the rotation of the articulated shaft 450, the support rod 3 may be turned over along with the rotation, thereby achieving the effect that the wheel structure forms a vertical angle, a horizontal angle, and the like with the ground by turnover. It should be noted that each of the articulated sheets A430 has a certain width. The width should be such that the sleeve 410 can rotate at 360° therebetween without coming into contact with the base 420, and both ends of the sleeve 410 can be inserted by the support rod 3.

In the present embodiment, two articulated sheets B440 corresponding to each other are arranged at the outer sides of the articulated sheets A430; the two articulated sheets B440 are fixedly connected with both ends of the articulated shaft 450 respectively; and the articulated sheets B440 are articulated relative to the articulated sheets A430 under the drive of the articulated shaft 450. In the present embodiment, each of the articulated sheets B440 is specifically semi-circular, which is only a preferred embodiment and may be in other shapes. The edges of the articulated sheets B440 are provided with a plurality of limiting grooves 460. The support rod 3 is provided with a limiting structure specifically composed of limiting blocks 321, a spring and a handle 320. A set of limiting blocks 321 corresponding to each other are arranged on the side wall of the support rod 3. The end of each limiting block 321 close to the support rod 3 is located in the support rod 3; the side wall of the support rod 3 in the position corresponding to the limiting block 321 is provided with a chute; and the limiting blocks 321 are embedded in the chute and may slide on the support rod 3 along the chute. The spring is arranged in the support rod 3; both ends of the spring are in the same direction as both ends of the support rod 3; the end of the spring close to the connecting piece 4 is connected with the end of each limiting block 321 located in the support rod 3; the limiting block 321 may be abutted against the top end of the chute under the action of the tension of the spring, that is, the limiting block 321 in this position may be clamped into the limiting groove 460 and then matched with the limiting groove and limited; and the articulated shaft 450 may not rotate under the action of limit, so that the purpose of matching with the turnover structure to limit is achieved. The handle 320 is arranged at one side of the support rod 3; the end of the handle 320 close to the support rod 3 is located in the support rod 3; the side wall of the support rod 3 in the position corresponding to the handle 320 is provided with a chute; the handle 320 is embedded in the chute and may slide on the support rod 3 along the chute; the end of the handle 320 in the support rod 3 is connected with the spring; and the spring may be compressed by pulling the handle 320 toward the direction of the wheel bodies 1, thereby driving the limiting blocks 321 to slide to the side away from the limiting groove 460. Thus, the matching limit of the limiting blocks 321 and the limiting groove 460 is released. When the handle 320 is released, under the action of the tension of the spring, the handle 320 is automatically reset to the position before pulling, and the limiting blocks 321 are also reset to the position when matched with the limit groove 460.

In the present embodiment, the kayak is provided with the above-mentioned wheel structure. The wheel structure is arranged at the stern of the kayak 6 by the connecting piece 4. If the kayak 6 needs to be loaded, the user can adjust the wheel direction by pressing the spring pin 310 and rotating the support rod 3. The user may separate the limiting blocks 321 from the limiting grooves 460 on the articulated sheets B440 by pulling the handle 320 on the support rod 3, and swing the support rod 3 clockwise or anti-clockwise in the direction of rotation of the articulated shaft 450 to turn over the support rod to an appropriate angle. At this angle, the limiting blocks 321 correspond to the limiting grooves 460, and then the handle 320 is released, so that the limiting blocks 321 are rebounded under the action of the spring and clamped into the limiting grooves 460 corresponding thereto to realize matching limit, thereby fixing the wheel structure at this angle and facilitating the loading of the kayak 6, as shown in Fig. 2. When the kayak 6 enters water, in order to prevent the wheel bodies 1 arranged at the stern from being immersed in the water, the wheel structure can be turned over upwards in the same operation as described above, so that the wheel bodies 1 are higher than the horizontal plane and may not be immersed in the water. This operation is easier than disassembling the wheel structure after entering the water and installing the wheel structure after going ashore.

In the present embodiment, as shown in Fig. 1, the base 420 is provided with sealing screws 470; the base 420 and the kayak 6 are installed by the sealing screws 470; and the sealing screws 470 can guarantee the sealing performance at the installing positions of the base 420 and the kayak 6 to prevent water leakage.

In the present embodiment, the support rod 3 is provided with a camber, and the specific structure is as follows: a section of the support rod 3 close to the wheel bodies 1 is provided with a camber and a section inserted into the sleeve 410 is straightly arranged. The reason for this arrangement is that: if the support rod 3 is entirely straight, when the support rod 3 is turned over to be parallel with the hull, the wheel bodies 1 may be come into contact with the hull, thereby affecting the rotation of the wheel bodies 1; and the problem can be avoided if a section of the support rod 3 is provided with a camber. The section inserted into the sleeve 410 is arranged straightly in order to avoid affecting the rotation of the support rod 3 in the sleeve 410 in the horizontal direction.

### Embodiment 2

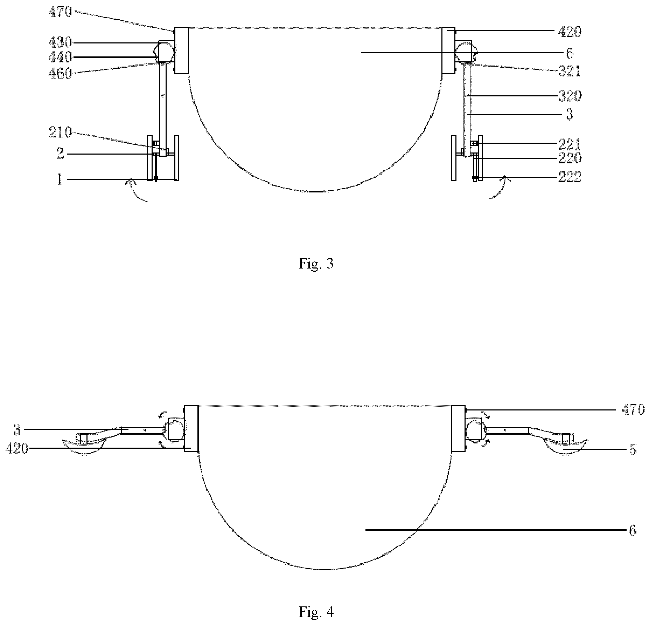

The present embodiment is different from embodiment 1 in that: in the present embodiment, as shown in Fig. 3, the wheel structures are arranged on both sides of the hull of the kayak 6 by the connecting piece 4. This connection mode is more suitable for the kayak 6 of which the stern has no sufficient position to connect with the base 420 but both sides of the hull have sufficient positions to connect with the base 420. The specific connection mode is that: the connecting piece 4 is arranged on both sides of the hull of the kayak 6 through the base 420, and the base 420 and the hull are installed by the sealing screws 470. The support rod 3 of the wheel structure is inserted into one end of the sleeve 410 of the connecting piece 4, and is movably connected with the connecting piece 4 through the limit of the spring pin 310 and the positioning holes 411, so that the wheel structures are installed on both sides of the kayak 6 by the connecting piece 4. When the user needs to move the kayak 6 onto the shore, the user only needs to turn over the wheel structure to be perpendicular to the ground in the mode in embodiment 1, and adjust the orientation of the wheel bodies 1 in the horizontal direction as needed, so that the kayak 6 can be moved on the shore through the wheel structure. After the kayak 6 enters water, the user can turn over the wheel structure in the same mode, so that the wheel bodies 1 may not be immersed in the water.

In the present embodiment, the sleeve 410 of the connecting piece 4 is detachably provided with a balanced buoy 5. The support rod 3 is arranged at one end of the balanced buoy 5, and the structure of the support rod 3 is the same as the wheel structure, that is, the only difference is that the balanced buoy is arranged at the other end of the support rod 3. Because the wheel structure is inserted into the sleeve 410 through the support rod 3 and realizes matching limit through the spring pin 310, that is, the wheel structure and the sleeve 410 are also detachably connected, the user can disassemble and replace the wheel structure with the balanced buoy 5 during use and turn over the wheel structure to be parallel with the water surface, as shown in Fig. 4. In this way, a kayak 6 provided with balanced buoys 5 oppositely on both sides of the hull is formed, and has better balance.

### Embodiment 3

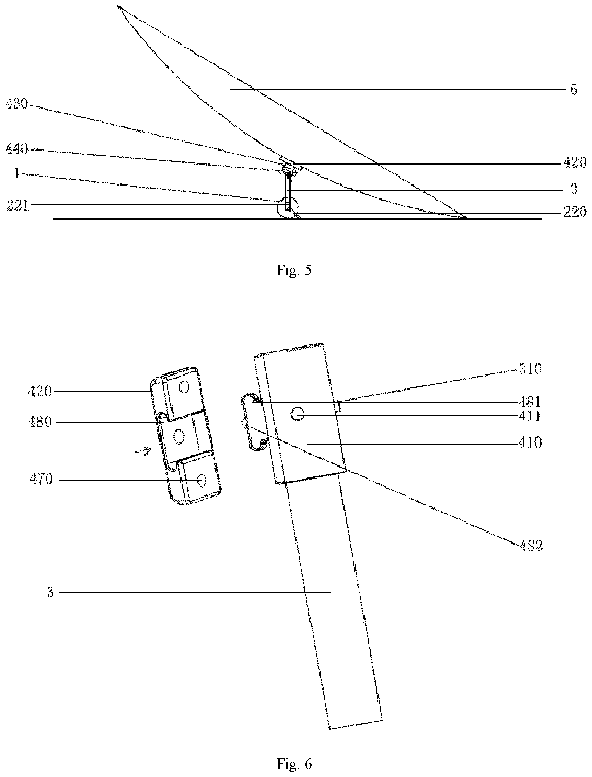

The present embodiment is different from embodiment 1 in that: in the present embodiment, the wheel structure is arranged at the bottom of the kayak 6 by the connecting piece 4, as shown in Fig. 5.

### Embodiment 4

The present embodiment is different from embodiment 1 in that: in the present embodiment, the connecting piece 4 is not provided with a turnover structure, and the connecting piece 4 is composed of a base 420 and a sleeve 410. The specific structure is that: a sliding block 481 is horizontally arranged at one side of the sleeve 410, the base 420 is horizontally provided with a chute 480, and by the matching of the sliding block 481 and the chute 480, the sleeve 410 is detachably connected with the base 420, as shown in Fig. 6. The side surface of the sleeve 410 is provided with a plurality of positioning holes 411 in the horizontal direction, and the positioning holes can be matched with the spring pin 310 of the support rod 3.

The wheel structure can be arranged at both sides or stern of the kayak 6; the base 420 is installed on the hull of the kayak 6 by sealing screws; the sleeve 410 is inserted into the support rod 3 and is matched with the spring pin 310 by the positioning holes 411 to realize limit; and if the user needs to disassemble the wheel structure from the kayak 6 or install the wheel structure on the kayak 6 during use, the user only needs to disassemble or install the sleeve 410 and the base 420, so that the operation is simple and convenient.

In the present embodiment, the sliding block 481 of the sleeve 410 is provided with a spring plunger 482; a ball head of the spring plunger 482 is arranged with respect to one side of the chute 480; and the inner wall of the chute 480 is provided with a recess correspondingly matched with the ball head of the spring plunger 482, so that when the sliding block 481 is inserted into the chute 480 and matched with the chute, the ball head of the spring plunger 482 may be bounced to be matched with the recess on the inner wall of the chute 480 to realize limit and fixation, thereby making the connection between the sleeve 410 and the base stabler. When the sleeve 410 and the base 420 need to be separated, because the ball head of the spring plunger 482 is spherical, the recess on the base 420 is matched with the ball head, and the inner wall is a curved slope surface. By applying a certain force to the sleeve 410 in the separating direction, the ball head of the spring plunger 482 is extruded by the slope surface of the recess and retracted, so that the limit of the ball head and the recess is released, thereby achieving disassembly.

In the present embodiment, the support rod 3 is not provided with the limiting blocks 321, the handle 320 and the spring.

Although preferred embodiments of the utility model are described in detail above, it should be clearly understood that for those skilled in the art, various variations and changes can be made to the utility model. Any modification, equivalent replacement and improvement made within the spirit and the principle of the utility model shall be included within the protection scope of the utility model.

## Claims

1. A wheel structure, having a main body composed of wheel bodies (1), a shaft (2) and a support rod (3), wherein a bidirectional ratchet wheel mechanism (210) is arranged between the shaft (2) and the support rod (3).

2. The wheel structure according to claim 1, wherein the shaft (2) is provided with a retreat preventing rod (220), and the retreat preventing rod (220) is movably arranged on the shaft (2).

3. The wheel structure according to claim 1, wherein the support rod (3) is provided with a camber.

4. A kayak, having a main body provided with any one of the above wheel structures of claims 1-3.

5. The kayaking according to claim 4, wherein the kayak (6) is provided with a connecting piece (4), and the wheel structure is connected with the kayak (6) by the connecting piece (4).

6. The kayak according to claim 5, wherein the connecting piece (4) is provided with a turnover structure, and the support rod (3) of the wheel structure is provided with a limiting structure matched with the turnover structure to realize limit.

7. The kayak according to claim 5, wherein the connecting piece (4) comprises a sleeve (410); the periphery of the sleeve (410) is provided with a plurality of positioning holes (411); and a spring pin (310) is arranged at one end of the support rod (3), and the spring pin (310) can be matched with the positioning holes (411) to realize limit.

8. The kayak according to claim 7, wherein the sleeve (410) is detachably provided with a balanced buoy (5).

9. The kayak according to claim 5, wherein the connecting piece (4) is formed by matching a base (420) with the sleeve (410); the base (420) is arranged on the main body of the kayak (6); the sleeve (410) is arranged at one end of the support rod (3); and the sleeve (410) is detachably connected with the base (420).

10. The kayak according to claim 9, wherein the base (420) and the main body of the kayak (6) are installed by sealing screws (470).
